Europäisches Patentamt

European Patent Office

Office européen des brevets

(11) Veröffentlichungsnummer: **0 296 406**

**A1**

# (12) EUROPÄISCHE PATENTANMELDUNG

(21) Anmeldenummer: **88109120.1**

(51) Int. Cl.4: **A61M 5/32**

(22) Anmeldetag: **08.06.88**

(30) Priorität: **19.06.87 IT 5344087**

(43) Veröffentlichungstag der Anmeldung:
**28.12.88 Patentblatt 88/52**

(84) Benannte Vertragsstaaten:
**AT BE CH DE ES FR GB IT LI NL SE**

(71) Anmelder: **EXPER-KG DES PERONI GUENTHER & CO.-S.A.S.**
**Reschenstrasse 20/D**
**I-39100 Bozen(IT)**

(72) Erfinder: **Macchiotti, Federico**
**via A. Saffi 2**
**I-10138 Torino(IT)**
Erfinder: **Angioi, Piergiorgio**
**via Ala di Stura 47**
**I-10148 Torino(IT)**
Erfinder: **Rossignoli, Cosimo**
**via della Vittoria 41**
**I-10147 Torino(IT)**
Erfinder: **Rossignoli, Carmine**
**piazza Statuto 12**
**I-10122 Torino(IT)**

(74) Vertreter: **Munk, Ludwig, Dipl.-Ing.**
**Patentanwalt Prinzregentenstrasse 1**
**D-8900 Augsburg(DE)**

(54) **Vorrichtung zur Entfernung von Schutzkappen und Kanülennadeln von Injektionsspritzen.**

(57) Die neue Vorrichtung umfaßt ein Traggestell (11, 12, 13), das mit elastischen Rückhalteteilen (23) versehen ist. Diese elastischen Rückhalteelemente (23) sind derart ausgelegt, daß sie sowohl mit der Schutzkappe (F) der Injektionsspritze (G) zur Bindung der Schutzkappe (F) an das Traggestell (11, 12, 13) als auch mit der Kanülennadel (H) zu deren Abzug von der Injektionsspritze (G) zusammenwirken können.

FIG. 4

# VORRICHTUNG ZUR ENTFERNUNG VON SCHUTZKAPPEN UND KANÜLENNADELN VON INJEKTIONSS-PRITZEN

Die vorliegende Erfindung betrifft eine Vorrichtung zur Entfernung von Kanülennadeln und Schutzkappen von Injektionsspritzen.

Bei der Verwendung von Injektionsspritzen und Blutabnahmen an Pazienten besteht bekannterweise für die Bedienungsperson die Gefahr durch die Injektionsspritze verletzt zu werden. Diese Verletzung könnte schwerwiegende Folgen für die Bedienungsperson haben, wenn beispielsweise die Kanülennadel zur Abnahme von Blut eines an einer ansteckenden Krankheit leidenden Patienten verwendet wurde.

Die Aufgabe der vorliegenden Erfindung liegt in der Schaffung einer Vorrichtung, die es erlaubt auf einfache und wirtschaftliche Weise den genannten Mangel zu beheben.

Erfindungsgemäß wird diese Aufgabe dadurch gelöst, daß die genannte Vorrichtung ein Traggestell umfaßt, das mit elastischen Rückhalteteilen versehen ist, die sowohl mit der Schutzkappe der Injektionsspritze zu deren elastischen Bindung an das Traggestell als auch mit der Kanülennadel zu deren Abzug von der Injektionsspritze zusammenwirken können.

Dank dieser Merkmale ist die Bedienungsperson imstande, die Vorgänge des Abzugs der Schutzkappe von der Kanülennadel, der Einführung der Kanülennadel in die Schutzkappe und des Abzugs der Kanülennadel und der Schutzkappe vom Injektionsspritzenkörper mit einer einzigen Hand auszuführen, ohne daß irgend ein Verletzungsrisiko bestünde.

Vorzugsweise umfassen die erwähnten elastischen Rückhalteteile ein Paar von im wesentlichen untereinander koplanaren, elastischen Lamellen, die auskragend am Tragegestell befestigt sind und mit einem Paar von zueinander zugekehrten, freien Rändern versehen sind.

Auf diese Art und Weise kann die Schutzkappe einer Injektionsspritze zwischen den erwähnten Rändern eingeführt werden, die dafür sorgen, daß sowohl die Schutzkappe in Stellung gehalten wird als auch zu verhindern, daß bei einem Auszug der Injektionsspritze nach der Einführung der Schutzkappe zwischen den genannten Rändern diese letztere zusammen mit der Kanülennadel ausgezogen wird.

Weitere Vorteile und Merkmale der erfindungsgemäßen Vorrichtung gehen näher aus der folgenden, eingehenden Beschreibung hervor, die beispielsweise unter Bezugnahme auf die anliegende Zeichnung erfolgt. Es zeigen

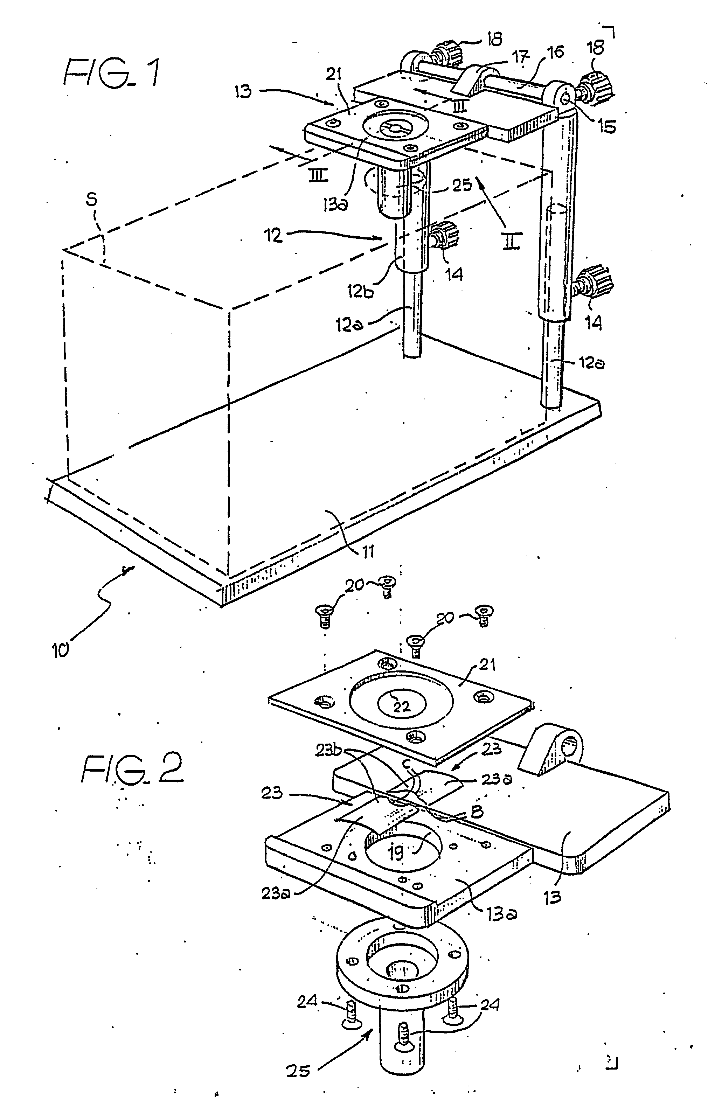

Figur 1 eine schaubildliche Ansicht einer erfindungsgemäßen Vorrichtung,

Figur 2 eine schaubildliche Explosionsansicht einer Einzelheit aus Figur 1,

Figur 3 eine Ansicht im Schnitt längs der Linie III-III aus Figur 1, und

Figur 4 eine teilweise geschnittene Seitenansicht der Vorrichtung aus Figur 1.

Unter Bezugnahme auf die Zeichnung, ist mit 10 in ihrer Gesamtheit eine Vorrichtung zur sicheren Entfernung von Schutzkappen und Kanülennadeln von Injektionsspritzen angegeben. Die Vorrichtung 10 umfaßt eine ebene Auflage 11, zwei vertikale Ständer 12, die im Bereich eines Randes der Auflage 11 befestigt sind, und eine Platte 13, die oben von den Ständern 12 getragen wird. Diese letzteren sind teleskopartig ausgeführt und umfassen jeweils eine festliegende Stange 12a, an der ein mit einer Rastschraube 14 versehener Rohrteil 12b verschiebbar angebracht ist.

Die Rohrteile 12b weisen im Bereich ihres oberen Endes durchgehende Querbohrungen 15 auf, in denen die Enden einer Lagerspindel 16 drehbar angebracht sind, auf der in 17 die Platte 13 befestigt ist. Die Drehung der Lagerspindel 16 in den Querbohrungen 15 kann auf für sich bekannte Weise mittels zweiter Rastschrauben 18 der Rohrteile 12b blockiert werden.

Die Platte 13 weist einen mit einer durchgehenden Bohrung 19 versehenen Abschnitt 13a auf. Auf der Oberfläche des Plattenabschnittes 13a der Platte 13 ist mittels Schrauben 20 eine Hilfssklemmplatte 21 befestigt, die in der Mitte mit einer durchgehenden, koaxialen und einen gegenüber der Bohrung 19 der Platte 13 kleineren Durchmesser aufweisenden Bohrung 22 versehen ist.

Zwischen der Platte 13 und der Hilfsplatte 21 sind zwei elastische Lamellen 23 derart verspannt, daß jede Lamelle 23 einen an der Platte 13 befestigten Abschnitt 23a und, im Bereich der Bohrung 19, einen Abschnitt 23b aufweist, der nach unten freielastisch biegsam ist, wobei die Biegung nach oben durch die Hilfsplatte 21 beschränkt oder verhindert wird.

An der unteren Fläche des Plattenabschnittes 13a der Platte 13 ist mittels Schrauben 24 eine Führungsprofilhülse 25 befestigt, die koaxial zu den Bohrungen 19 und 22 angeordnet ist.

Im Bereich der entsprechenden, freien Abschnitte 23b weisen die elastischen Lamellen 23 gegenüberliegende Ränder B auf, die mit halbkreisförmigen Ausnehmungen C versehen sind, deren Wirkungsweise aus der folgenden Beschreibung hervorgeht.

In der Arbeitsstellung der Vorrichtung 10 wird auf die Auflage 11 ein Sammelbehälter S gegeben.

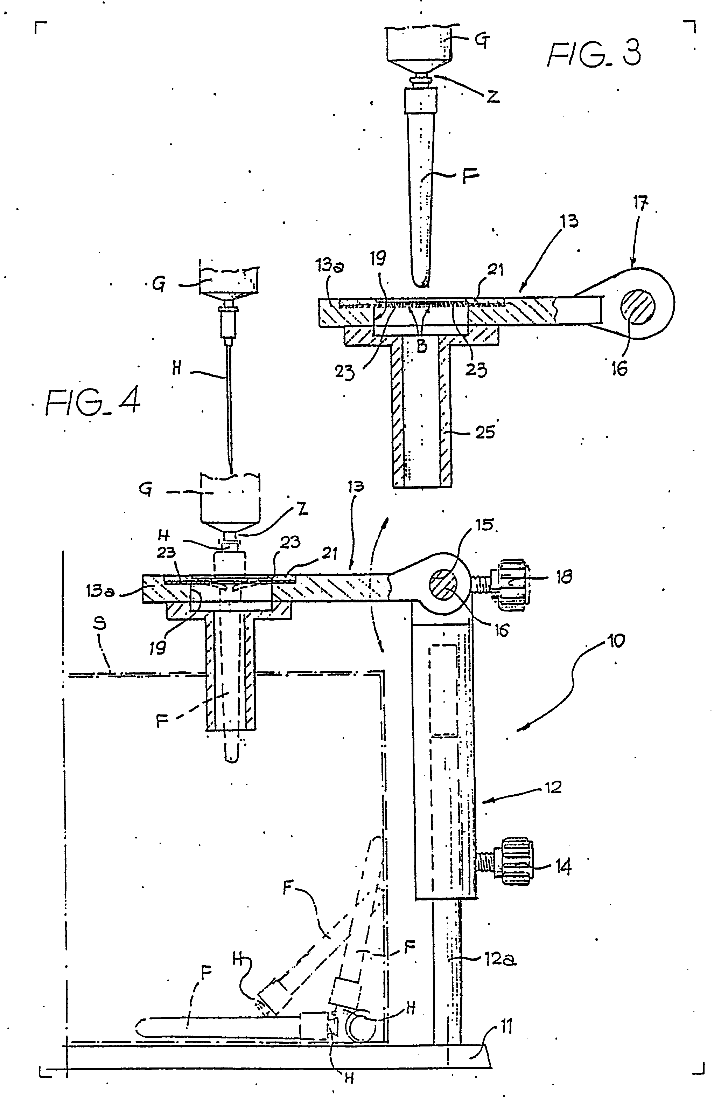

Nachfolgend kann die Bedienungsperson gegebenenfalls die Stellung der Platte 13 gegenüber dem Sammelbehälter S einstellen, indem sie auf die Rastschrauben 14 und 18 der Rohrteile 12b der teleskopartigen Ständer 12 einwirkt. Muß nun die Bedienungsperson eine Schutzkappe von der Injektionsspritze F bzw. G abziehen, so wird bei Handhabung der Injektionsspritze G die Schutzkappe F in die Bohrung 22 der Hilfsplatte 21 soweit eingesetzt, bis der elastische Eingriff zwischen den Rändern B der Lamellen 23 und der Schutzkappe im Bereich der Ausnehmungen C hervorgerufen wird. Bei diesem Eingriff werden die Abschnitte 23b der Lamellen 23 nach unten gebogen; zieht nachfolgend die Bedienungsperson die Injektionsspritze G aus der Vorrichtung 10, wird die Schutzkappe F durch die Ränder B der Lamellen 23 zurückgehalten (Figur 4) und die Bedienungsperson kann die Injektionsspritze mit der freiliegenden Nadel H benützen.

Am Ende dieser Benützung führt die Bedienungsperson die Kanülennadel H in die zwischen den Lamellen 23 verklemmte Schutzkappe F ein. Nach der erfolgten Einführung übt die Bedienungsperson auf die Injektionsspritze G eine nach unten gerichtete Kraft derart aus, daß die elastischen Lamellen 23 weiter aufgespreizt werden bis diese letzteren nach einer Gleitbewegung auf der Oberfläche der Schutzkappe F sich schnappartig im zwischen dem Ansatz der Kanülennadel H und der Injektionsspritze G liegenden Bereich ausrichten, Bereich, der in der Zeichnung mit dem Bezugszeichen Z angegeben ist. In dieser Stellung verursacht ein auf die Injektionsspritze G ausgeübte Zugkraft den Abzug der Kanülennadel H und der entsprechenden Schutzkappe F vom Ansatz der Injektionsspritze selbst, wobei nachfolgend die Kanülennadel und die Schutzkappe in den Sammelbehälter S fallen. Bei dieser Zugeinwirkung auf die Injektionsspritze G kommt nämlich der Ansatz der Kanülennadel H zum Anschlag auf die untere Fläche der Lamellen 23, die wegen ihres Anschlages auf der Hilfsplatte 21 nicht nach oben gebogen werden können. Es versteht sich, daß die Wirkungsweise der vorliegenden Erfindung sich auf alle Vorrichtungen erstreckt, die dieselbe Nützlichkeit bei Verwendung desselben Erfindungsgedanken erzielen.

## Ansprüche

1. Vorrichtung zur Entfernung von Kanülennadeln und Schutzkappen von Injektionsspritzen, **dadurch gekennzeichnet,** daß sie ein Traggestell (11, 12, 13) umfaßt, das mit elastischen Rückhalteteilen (23) versehen ist, die imstande sind sowohl mit der Schutzkappe (F) der Injektionsspritze (G) zur Bindung der Schutzkappe an das Traggestell (11, 12, 13) als auch mit der Kanülennadel (H) zu deren Abzug von der Injektionsspritze (G) zusammen zu wirken.

2. Vorrichtung gemäß Anspruch 1, **dadurch gekennzeichnet,** daß die erwähnten elastischen Rückhalteteile ein Paar von im wesentlichen in derselben Ebene liegenden, elastischen Lamellen (23) umfassen, die auskragend am Traggestell (13a, 21) befestigt sind und mit einem Paar freier, gegenüberliegender Ränder (B) versehen sind.

3. Vorrichtung nach Anspruch 2, **dadurch gekennzeichnet,** daß die erwähnten freien Ränder (B) Profilausnehmungen (C) aufweisen.

4. Vorrichtung nach einer der vorangehenden Ansprüchen, **dadurch gekennzeichnet,** daß das Traggestell (11, 12, 13) eine mit einer durchgehenden Bohrung (19) versehene Platte aufweist, wobei im Bereich dieser durchgehenden Bohrung (19) die erwähnten elastischen Rückhalteteile (23) befestigt sind.

5. Vorrichtung nach Anspruch 4, **dadurch gekennzeichnet,** daß das Traggestell überdies eine Auflage (11) umfaßt, die zumindest einen höhenverstellbaren, vertikalen Ständer (12) besitzt, mit dem die erwähnte Platte (13) verbunden ist.

6. Vorrichtung nach Anspruch 5, **dadurch gekennzeichnet,** daß die Platte (13) am Ständer (12) angelenkt ist und dadurch, daß Mittel (18) zur Festlegung der Stellung der Platte (13) am Ständer (12) vorgesehen sind.

7. Vorrichtung nach Anspruch 2 und 4, **dadurch gekennzeichnet,** daß das Traggestell eine gelochte Hilfsplatte (21) aufweist, die an der oben erwähnten Platte (13) und der Zwischenschaltung des Paares von elastischen Lamellen (23) befestigt ist.

8. Vorrichtung nach Anspruch 7, **dadurch gekennzeichnet,** daß an der Platte (13), auf der zur Hilfsplatte (21) abgewandten Seite, ein Rohrteil (25) zur Führung der Schutzkappe (F) befestigt ist.

FIG. 1

FIG. 2

FIG. 3

FIG. 4

Europäisches
Patentamt

EUROPÄISCHER RECHERCHENBERICHT

Nummer der Anmeldung

EP 88 10 9120

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 160 849 (MAYER) <br> * Ansprüche 3,5,6,8; Figuren 5-7 * | 1 | A 61 M 5/32 |
| A | | 2,3,8 | |
| A | GB-A-2 040 268 (ELISHA) <br> * Ansprüche 1,5-7,12; Figuren 2,3,5,6 * | 1-3 | |
| A | DE-A-2 740 335 (MALLICHE) <br> * Ansprüche 1-3,9; Seite 19, Zeilen 12-23; Figuren 1-3,11,12 * | 1-3 | |
| A | US-A-4 520 926 (NELSON) <br> * Anspruch 1; Figur 1 * | 1-3 | |
| A | FR-A-2 490 486 (SEDAN-SEIDMAN) <br> * Ansprüche 1,2; Seite 3, Zeilen 26-33; Figur 1 * | 5 | |

**RECHERCHIERTE SACHGEBIETE (Int. Cl.4)**

A 61 M 5/00

Der vorliegende Recherchenbericht wurde für alle Patentansprüche erstellt

| Recherchenort | Abschlußdatum der Recherche | Prüfer |
|---|---|---|
| BERLIN | 20-09-1988 | PAPA E.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)